# NEW EUROPEAN PATENT SPECIFICATION

(11) **EP 0 383 779 B2**
(45) Date of publication and mention of the opposition decision: **31.05.2000**
(45) Mention of the grant of the patent: 08.12.1993
(21) Application number: 88908169.1
(22) Date of filing: 02.09.1988
(51) Int. Cl.: C12N 15/00, C12N 1/14, C12P 21/02, C12R 1/66

(54) **PROCESS FOR THE PRODUCTION OF PROTEIN PRODUCTS IN $i(ASPERGILLUS) AND PROMOTERS FOR USE IN $i(ASPERGILLUS)**
VERFAHREN ZUR HERSTELLUNG VON PROTEINPRODUKTEN IN $i(ASPERGILLUS) UND PROMOTOREN ZUR VERWENDUNG IN $i(ASPERGILLUS)
PROCEDE DE PRODUCTION DE PROTEINES DANS $i(ASPERGILLUS) ET PROMOTEURS DESTINES A EXPRIMER CE CHAMPIGNON

(30) Priority: 04.09.1987 DK 460987; 29.09.1987 DK 512687
(43) Date of publication of application: 29.08.1990
(73) Proprietor: NOVO NORDISK A/S, 2880 Bagsvaerd (DK)
(72) Inventor: WÖLDIKE, Helle, Fabricius, DK-3540 Lynge (DK)
(74) Representative: Bassett, Richard Simon
(86) International application number: DK8800145
(87) International publication number: WO8901969

(56) References cited:
- EP-A- 0 191 221
- EP-A- 0 215 594
- WO-A-86/06097

## Description

The present invention relates to a process for expression of protein products in Aspergillus, recombinant DNA vectors, a promoter for Aspergillus and transformed fungi.

In the past, numerous processes have been developed for the production of polypeptides or proteins by means of the recombinant DNA technology. The main interest has been concentrated on bacteria and yeast, e.g. E. coli, Bacillus subtilis and Saccharomyces cerevisiae being well characterized species as regards for instance expression and selection systems.

Besides the above mentioned microorganisms, filamentous fungi, such as Aspergillus niger, are attractive candidates as host microorganisms for recombinant DNA vectors being well-characterized and widely used microorganisms for the commercial production of enzymes. Efforts have especially been concentrated on the development of transformation systems by which a selection marker permitting selection of transformants from the untransformed host microorganisms is used.

In the last few years different selection markers for the transformation of Aspergillus nidulans have been described and procedures have been developed for integrative transformation of the filamentous fungus Aspergillus nidulans for the purpose of investigation of the genetic and molecular processes controlling fungal cell differentiation.

Transformation of A. nidulans has been demonstrated by using plasmids containing the Neurospora crassa pyr-4 gene (Ballance, D.J. et al., Biochem.Biophys. Res.Commun., 112 (1983) 284-289), the A. nidulans amdS gene (Tilburn, J.G. et al., Gene 26 (1983) 205-221), the A. nidulans trpC gene (Yelton, M.M. et al., Proc.Natl. Acad.Sci. U.S.A., 81 (1984) 1470-1474) and the A. nidulans argB gene (John, M.A. and Peberdy J., Microb.Technol. 6 (1984) 386-389). The transforming DNA was found to be integrated into the host genome at rather low frequencies (typically < 1000 transformants/µg of DNA).

Recently transformation of Aspergillus niger with the amdS gene of A. nidulans was described (Kelly, J.M. and Hynes, M.J., EMBO Journal 4 (1985), 475-479) and amdS was shown to be a potential selection marker for use in transformation of Aspergillus niger that cannot grow strongly on acetamide as a sole nitrogen source. Transformation of Aspergillus niger using the argB gene of Aspergillus nidulans has also been described recently (Buxton, F. P. et al., Gene 37 (1985), 207-214).

So far yields of heterologous proteins have not been satisfactory in A. niger for commercial production. Accordingly, it is the object of the present invention to provide a method for obtaining commercially attractive yields of foreign proteins in Aspergillus. It is also an object of the present invention to enhance the production of homologous proteins in Aspergillus.

### BRIEF DESCRIPTION OF THE INVENTION

According to the present invention it has now been shown that it is possible to obtain a high level of expression of heterologous proteins or to enhance the production of homologous proteins in Aspergillus when using promoters derived from amylase genes from A. niger.

As used herein the expression "heterologous proteins" means proteins not produced by the host organism whereas "homologous proteins" means proteins produced by the host organism.

According to a first aspect of the present invention there is provided promoter and upstream activating sequences usable for Aspergillus, especially A. niger expression and derived from an A. niger neutral α-amylase gene.

According to a second aspect of the present invention there is provided promoter and upstream activating sequences derived from a previously undescribed amylase from A. niger (A. niger XA amylase).

The neutral and acid stable α-amylases from A. niger are described by Minoda et al., Agr.Biol.Chem. 33 (4), 572-578 (1969).

According to a third aspect of the present invention there is provided a process for expression of a protein product in Aspergillus comprising the steps of:
(a) providing a recombinant DNA cloning vector system capable of integration into the genome of an Aspergillus host in one or more copies and comprising: promoter and upstream activating sequences derived from an A. niger amylase gene; a suitable marker for selection of transformants; and a DNA-sequence encoding the desired protein product;
(b) transforming the Aspergillus host which does not harbour a functional gene for the chosen selection marker with the recombinant DNA cloning vector system from step a; and
(c) culturing the transformed Aspergillus host in a suitable culture medium.

The host strain is preferably an Aspergillus niger strain although other Aspergillus strains may be used.

According to a fourth aspect of the present invention there is provided a method for production of a protein product in Aspergillus niger by which method an Aspergillus niger strain being transformed with a recombinant DNA cloning vector system as described above is cultured in a suitable culture medium and the product is recovered from the culture medium.

### BRIEF DESCRIPTION OF THE DRAWINGS:

The present invention is further illustrated by reference to the accompanying drawings in which:
Fig. 1 shows an endonuclease restriction map of plasmids pNA1 and pNA2,
Fig. 2 shows the DNA-sequence of the A. niger neutral α-amylase promoter NA1 and upstream activating regions, the preregion and the 5'part of the structural gene for the A. niger neutral α-amylase,
Fig. 3 shows the DNA-sequence of the A. niger neutral α-amylase promoter NA2 and upstream activating sequences, the preregion and the 5' part of the structural gene for the A. niger neutral α-amylase.
Fig. 4 shows the construction of plasmid pXA,
Fig. 5 shows the DNA-sequence of the XA niger amylase promoter and upstream activating sequences together with the preregion and the 5' part of the structural gene,
Fig. 6 shows plasmid pAA,
Fig. 7a and b show the DNA-sequence of the acid-stable α-amylase promoter and upstream activating sequences together with the preregion and the 5' part of the structural gene,
Fig. 8 shows the construction of plasmid pNA2-RMP,
Fig. 9 shows the construction of plasmid pPAA-RMP
   and
Fig. 10 shows the construction of plasmids pPXA-RMP and pPXA-RMP'

### DETAILED DESCRIPTION OF THE INVENTION

The transformation technique used was a method adapted from the methods for transformation of A. nidulans (Ballance et al. Biochem.Biophys.Res.Commun., 112 (1983), 284-289; Tilburn et al., Gene 26 (1983), 205-221, Yelton et al. Proc.Natl.Acad.Sci. USA, 81 (1984), 1470-1474) and similar to the method of Buxton et al. (Gene 37 (1985), 207-214) for transformation of A. niger. In the process of the present invention the chosen Aspergillus strain is transformed with a vector system containing a selection marker which is capable of being incorporated into the genome of the host strain, but which is not harboured in the host strain before the transformation. Transformants can then be selected and isolated from nontransformants on the basis of the incorporated selection marker.

Preferred selection markers are the argB (A. nidulans or A. niger), trpC (A. nidulans), amdS (A. nidulans), or pyr4 (Neurospora crassa) genes, or the DHFR (dihydrofolate reductase or mutants hereof) gene. More preferred selection markers are the argB or the amdS gene.

Besides promoter and upstream activating sequences the vectors will normally contain further DNA-sequences encoding functions facilitating gene expression such as transcription terminators and polyadenylation signals.

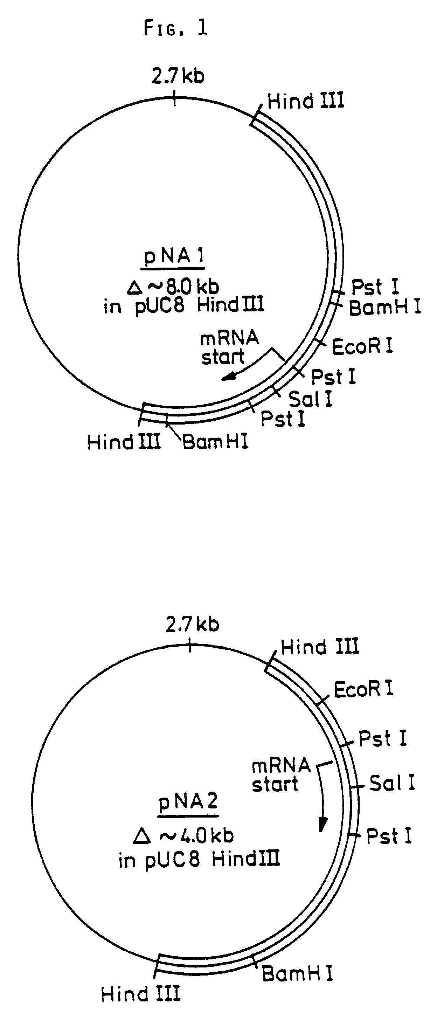

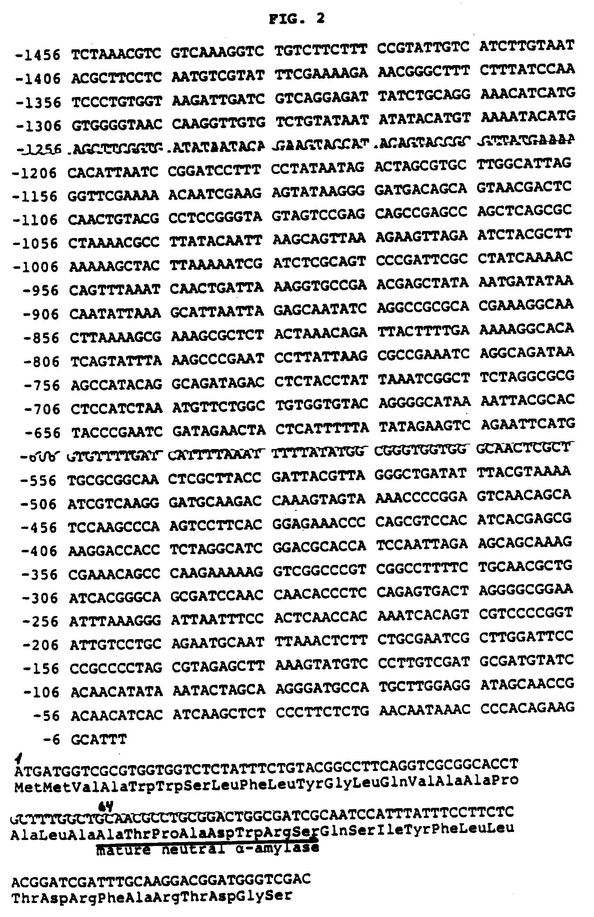

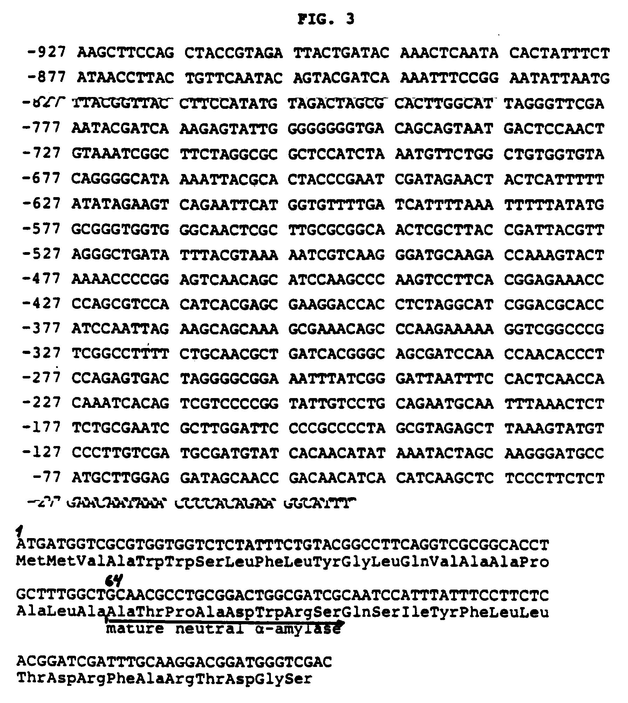

As described in further detail in example 1 DNA-sequences encoding the A. niger neutral α-amylase including the preregion and promoter and upstream activating sequences were derived from a A. niger mycelium and inserted into HindIII digested pUC8 to give plasmids pNA1 and pNA2 (see Fig. 1). In pNA1 the A. niger derived DNA is shown as a 8.0 kb HindIII-HindIII fragment. The established DNA-sequence of the promoter and upstream activating sequences is shown in Fig. 2. The promoter ends at nucleotide-1 preceding the Met(1) codon of the neutral α-amylase presequence. The nucleotide sequence encoding the presequence is constituted of 63 nucleotides and the mature α-amylase starts at a position corresponding to nucleotide 64. In pNA2 the A. niger derived DNA is shown as a 4.0 kb HindIII-HindIII fragment. The established DNA-sequence of the promoter and upstream activating sequences is shown in fig. 3. The promoter ends at nucleotide-1 preceding the Met(1) codon of the α-amylase presequence. The nucleotide sequence encoding the presequence is constituted of 63 nucleotides and the mature neutral α-amylase starts at a position corresponding to nucleotide 64.

From pNA1 and pNA2 the whole promoter sequence including sequences upstream to the promoter or functional parts thereof may be derived by means evident to the person skilled in the art. The promoter sequence may be provided with linkers with the purpose of introducing specific restriction sites facilitating the ligation of the promoter sequence with further DNA, for instance the gene encoding the desired protein product or different preregions (signal peptides).

According to one embodiment of the present invention the NA1 promoter and upstream activating sequences have the following sequence or a functionally equivalent nucleotide sequence: 
TCTAAAC[?]TC GTCAAAGGTC TGTCTTCTTT CCGTATTGTC ATCTTGTAAT
ACGCTTCCTC AATGTCGTAT TTCGAAAAGA AACGGGCTTT CTTTATCCAA
TCCCTGTGGT AAGATTGATC GTCAGGAGAT TATCTGCAGG AAACATCATG
GTGGGGTAAC CAAGGTTGTG TCTGTATAAT ATATACATGT AAAATACATG
AGCTTCGGTG ATATAATACA GAAGTACCAT ACAGTACCGC GTTATGAAAA
CACATTAATC CGGATCCTTT CCTATAATAG ACTAGCGTGC TTGGCATTAG
GGTTCGAAAA ACAATCGAAG AGTATAAGGG GATGACAGCA GTAACGACTC
CAACTGTACG CCTCCGGGTA GTAGTCCGAG CAGCCGAGCC AGCTCAGCGC
CTAAAACGCC TTATACAATT AAGCAGTTAA AGAAGTTAGA ATCTACGCTT
AAAAAGCTAC TTAAAAATCG ATCTCGCAGT CCCGATTCGC CTATCAAAAC
CAGTTTAAAT CAACTGATTA AAGGTGCCGA ACGAGCTATA AATGATATAA
CAATATTAAA GCATTAATTA GAGCAATATC AGGCCGCGCA CGAAAGGCAA
CTTAAAAGCG AAAGCGCTCT ACTAAACAGA TTACTTTTGA AAAAGGCACA
TCAGTATTTA AAGCCCGAAT CCTTATTAAG CGCCGAAATC AGGCAGATAA
AGCCATACAG GCAGATAGAC CTCTACCTAT TAAATCGGCT TCTAGGCGCG
CTCCATCTAA ATGTTCTGGC TGTGGTGTAC AGGGGCATAA AATTACGCAC
TACCCGAATC GATAGAACTA CTCATTTTTA TATAGAAGTC AGAATTCATG
GTGTTTTGAT CATTTTAAAT TTTTATATGG CGGGTGGTGG GCAACTCGCT
TGCGCGGCAA CTCGCTTACC GATTACGTTA GGGCTGATAT TTACGTAAAA
ATCGTCAAGG GATGCAAGAC CAAAGTAGTA AAACCCCGGA GTCAACAGCA
TCCAAGCCCA AGTCCTTCAC GGAGAAACCC CAGCGTCCAC ATCACGAGCG
AAGGACCACC TCTAGGCATC GGACGCACCA TCCAATTAGA AGCAGCAAAG
CGAAACAGCC CAAGAAAAAG GTCGGCCCGT CGGCCTTTTC TGCAACGCTG
ATCACGGGCA GCGATCCAAC CAACACCCTC CAGAGTGACT AGGGGCGGAA
ATTTAAAGGG ATTAATTTCC ACTCAACCAC AAATCACAGT CGTCCCCGGT
ATTGTCCTGC AGAATGCAAT TTAAACTCTT CTGCGAATCG CTTGGATTCC
CCGCCCCTAG CGTAGAGCTT AAAGTATGTC CCTTGTCGAT GCGATGTATC
ACAACATATA AATACTAGCA AGGGATGCCA TGCTTGGAGG ATAGCAACCG
ACAACATCAC ATCAAGCTCT CCCTTCTCTG AACAATAAAC CCCACAGAAG
GCATTT
 representing the sequence from nucleotide -1456 to -1 in Fig. 2.

According to a further embodiment the NA2 promoter and upstream activating sequences have the following sequence or a functionally equivalent nucleotide sequence: 
AAGCTTCCAG CTACCGTAGA TTACTGATAC AAACTCAATA CACTATTTCT
ATAACCTTAC TGTTCAATAC AGTACGATCA AAATTTCCGG AATATTAATG
TTACGGTTAC CTTCCATATG TAGACTAGCG CACTTGGCAT TAGGGTTCGA
AATACGATCA AAGAGTATTG GGGGGGGTGA CAGCAGTAAT GACTCCAACT
GTAAATCGGC TTCTAGGCGC GCTCCATCTA AATGTTCTGG CTGTGGTGTA
CAGGGGCATA AAATTACGCA CTACCCGAAT CGATAGAACT ACTCATTTTT
ATATAGAAGT CAGAATTCAT GGTGTTTTGA TCATTTTAAA TTTTTATATG
GCGGGTGGTG GGCAACTCGC TTGCGCGGCA ACTCGCTTAC CGATTACGTT
AGGGCTGATA TTTACGTAAA AATCGTCAAG GGATGCAAGA CCAAAGTACT
AAAACCCCGG AGTCAACAGC ATCCAAGCCC AAGTCCTTCA CGGAGAAACC
CCAGCGTCCA CATCACGAGC GAAGGACCAC CTCTAGGCAT CGGACGCACC
ATCCAATTAG AAGCAGCAAA GCGAAACAGC CCAAGAAAAA GGTCGGCCCG
TCGGCCTTTT CTGCAACGCT GATCACGGGC AGCGATCCAA CCAACACCCT
CCAGAGTGAC TAGGGGCGGA AATTTATCGG GATTAATTTC CACTCAACCA
CAAATCACAG TCGTCCCCGG TATTGTCCTG CAGAATGCAA TTTAAACTCT
TCTGCGAATC GCTTGGATTC CCCGCCCCTA GCGTAGAGCT TAAAGTATGT
CCCTTGTCGA TGCGATGTAT CACAACATAT AAATACTAGC AAGGATGCC
ATGCTTGGAG GATAGCAACC GACAACATCA CATCAAGCTC TCCCTTCTCT
GAACAATAAA CCCCACAGAA GGCATTT
 representing the sequence from nucleotide -927 to -1 in Fig. 3.

When comparing the NA1-sequence with the NA2-sequence it appears that they have almost identical sequences in part of the upstream activating region. These sequences extend up to nucleotide -725 and further from nucleotide -1129 to -1099 in fig. 2 and from nucleotide -755 to -725 in fig. 3.

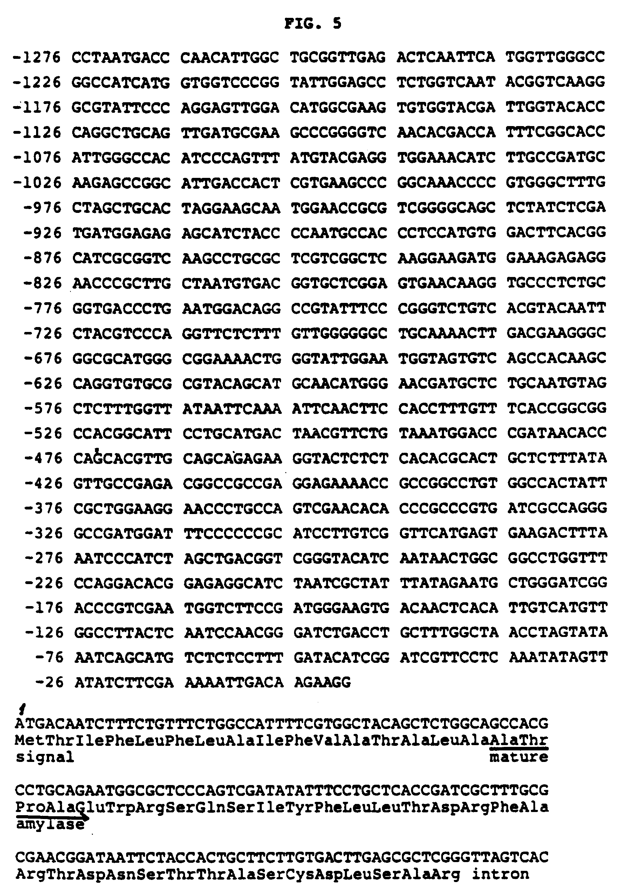

According to a still further embodiment of the present invention the A. niger XA derived promoter and upstream activating sequences have the following nucleotide sequence 
CCTAATGACC CAACATTGGC TGCGGTTGAG ACTCAATTCA TGGTTGGGCC
GGCCATCATG GTGGTCCCGG TATTGGAGCC TCTGGTCAAT ACGGTCAAGG
GCGTATTCCC AGGAGTTGGA CATGGCGAAG TGTGGTACGA TTGGTACACC
CAGGCTGCAG TTGATGCGAA GCCCGGGGTC AACACGACCA TTTCGGCACC
ATTGGGCCAC ATCCCAGTTT ATGTACGAGG TGGAAACATC TTGCCGATGC
AAGAGCCGGC ATTGACCACT CGTGAAGCCC GGCAAACCCC GTGGGCTTTG
CTAGCTGCAC TAGGAAGCAA TGGAACCGCG TCGGGGCAGC TCTATCTCGA
TGATGGAGAG AGCATCTACC CCAATGCCAC CCTCCATGTG GACTTCACGG
CATCGCGGTC AAGCCTGCGC TCGTCGGCTC AAGGAAGATG GAAAGAGAGG
AACCCGCTTG CTAATGTGAC GGTGCTCGGA GTGAACAAGG TGCCCTCTGC
GGTGACCCTG AATGGACAGG CCGTATTTCC CGGGTCTGTC ACGTACAATT
CTACGTCCCA GGTTCTCTTT GTTGGGGGGC TGCAAAACTT GACGAAGGGC
GGCGCATGGG CGGAAAACTG GGTATTGGAA TGGTAGTGTC AGCCACAAGC
CAGGTGTGCG CGTACAGCAT GCAACATGGG AACGATGCTC TGCAATGTAG
CTCTTTGGTT ATAATTCAAA ATTCAACTTC CACCTTTGTT TCACCGGCGG
CCACGGCATT CCTGCATGAC TAACGTTCTG TAAATGGACC CGATAACACC
CAGCACGTTG CAGCAGAGAA GGTACTCTCT CACACGCACT GCTCTTTATA
GTTGCCGAGA CGGCCGCCGA GGAGAAAACC GCCGGCCTGT GGCCACTATT
CGCTGGAAGG AACCCTGCCA GTCGAACACA CCCGCCCGTG ATCGCCAGGG
GCCGATGGAT TTCCCCCCGC ATCCTTGTCG GTTCATGAGT GAAGACTTTA
AATCCCATCT AGCTGACGGT CGGGTACATC AATAACTGGC GGCCTGGTTT
CCAGGACACG GAGAGGCATC TAATCGCTAT TTATAGAATG CTGGGATCGG
ACCCGTCGAA TGGTCTTCCG ATGGGAAGTG ACAACTCACA TTGTCATGTT
GGCCTTACTC AATCCAACGG GATCTGACCT GCTTTGGCTA ACCTAGTATA
AATCAGCATG TCTCTCCTTT GATACATCGG ATCGTTCCTC AAATATAGTT
ATATCTTCGA AAAATTGACA AGAAGG
 or a functionally equivalent sequence. This sequence represents the sequence from nucleotide -1 to -1276 in fig. 5.

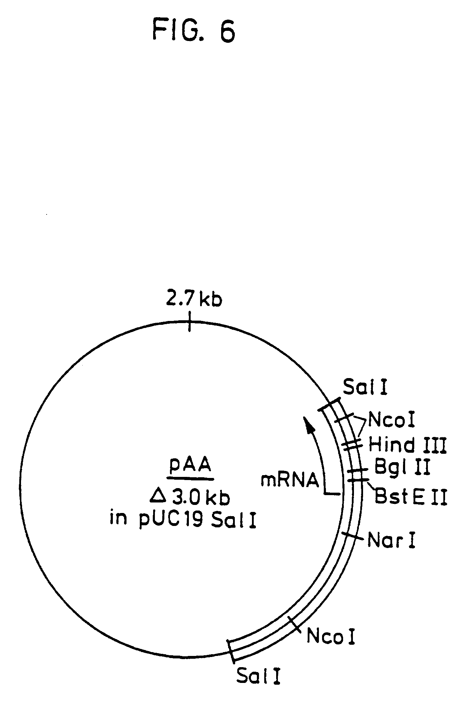

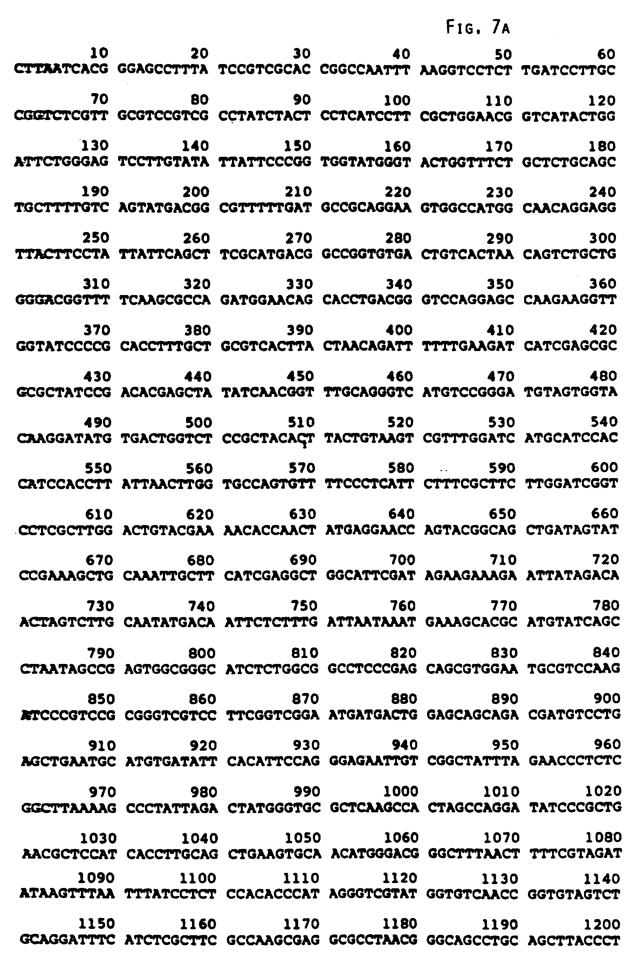

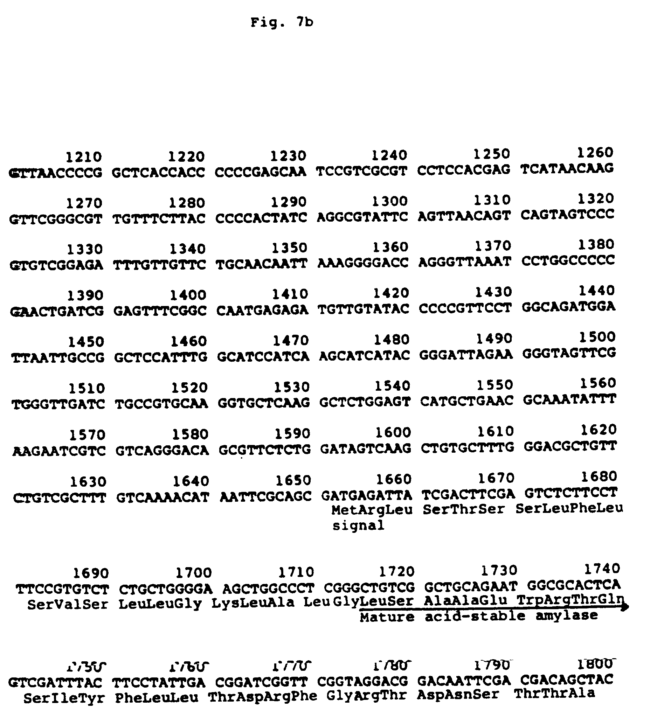

The A. niger acid stable α-amylase promoter may be derived from plasmid pAA (fig. 6 and example 3). The promoter and upstream activating sequences are included in the SalI-BstEII fragment of pAA. The DNA-sequence of the promoter including upstream activating sequences together with the 5' part of the mature acid-stable α-amylase gene is shown in fig. 7a and b.

The present invention is contemplated to include use of some of the above indicated sequences or functional parts or fragments thereof.

The terminators and polyadenylation sequences may be derived from the same sources as the promoters. Enhancer sequences may also be inserted into the construction.

The expressed product may be accumulated within the cells requiring disruption of the cells to isolate the product. To avoid this further process step and also to minimize the amount of possible degradation of the expressed product within the cells it is preferred that the product is secreted from the cells. For this purpose the gene for the desired product is provided with a preregion ensuring effective direction of the expressed product into the secretory pathway of the cell. This preregion which might be a naturally occuring signal or leader peptide or functional parts thereof or a synthetic sequence providing secretion is generally cleaved from the desired product during secretion leaving the mature product ready for isolation from the culture broth.

The preregion may be derived from genes for secreted proteins from any source of organism.

According to the present invention the preregion may be derived from a glucoamylase or an amylase gene from an Aspergillus species, an amylase gene from a Bacillus species, a lipase or proteinase gene from Rhizomucor miehei, the gene for the α-factor from S. cerevisiae, the calf prochymosin gene or from the gene for the protein product to be produced by the transformed strain. More preferably the preregion is is derived from the gene for A. oryzae TAKA amylase, A. niger neutral α-amylase, A. niger acid-stable α-amylase, preregion from XA amylase, B. licheniformis α-amylase, the maltogenic amylase from Bacillus NCIB 11837, B. stearothermophilus α-amylase or B. licheniformis subtilisin.

The TAKA-amylase signal and the A. niger neutral α-amylase signal have the following sequence

ATGATGGTCGCGTGGTGGTCTCTATTTCTGTACGGCCTTCAGGTCGCGGCACCT
MetMetValAlaTrpTrpSerLeuPheLeuTyrGlyLeuGlnValAlaAlaPro

GCTTTGGCT
AlaLeuAla

The gene for the desired product functionally linked to the promoter and terminator sequences may be incorporated in a vector containing the selection marker or may be placed on a separate vector or plasmid capable of being integrated into the genome of the host strain. As used herein the expression "vector system" includes a single vector or plasmid or two or more vectors or plasmids which together contain the total DNA-information to be integrated into the host genome. Vectors or plasmids may be linear or closed circular molecules. According to a preferred embodiment of the present invention A. niger is cotransformed with two vectors, one including the selection marker and the other comprising the remaining foreign DNA to be introduced in the host strain, including promoter, the gene for the desired product and transcription terminator and polyadenylation sequences.

Normally the A. niger transformants are stable and can be cultured in the absence of a selection pressure. If the transformants turn out to be unstable the selection marker may be used for selection during cultivation. The transformed cells are then cultured under a selection pressure corresponding to the marker in question.

The present invention provides for a method for production of high yields of many different polypeptide or protein products in Aspergillus, especially A. niger. A. niger strains have for years been used in commercial scale for the production of for instance amyloglucosidase and other extracellular enzymes and accordingly fermentation technology for these microorganisms is well developed and the microorganisms are approved for use in the food industry. The present invention offers the possibility of using A. niger in the industrial production of high amounts of in principle any polypeptide or protein product. Examples of such products are chymosin or prochymosin and other rennets, proteases, amyloglucosidases, acid stable amylases from Aspergillus, fungal lipases or prokaryotic lipases, and thermostable bacterial and fungal amylases.

The genes for these enzymes were obtained from cDNA libraries or genomic libraries as described in further detail in the following.

The cloning of a gene for this previously undescribed amylase is described in the following example 2. From the DNA-sequence for the gene for the mature amylase (XA) the following amino acid sequence was deduced for the novel amylase:

Ala-Thr-Pro-Ala-Glu-Trp-Arg-Ser-Gln-Ser-Ile-Tyr-Phe-Leu-
Leu-Thr-Asp-Arg-Phe-Ala-Arg-Thr-Asp-Asn-Ser-Thr-Thr-Ala-
Ser-Cys-Asp-Leu-Ser-Ala-Arg-Gln-Tyr-Cys-Gly-Gly-Ser-Trp-
Gln-Gly-Ile-Ile-Asn-Gln-Leu-Asp-Tyr-Ile-Gln-Gly-Met-Gly-
Phe-Thr-Ala-Ile-Trp-Ile-Thr-Pro-Val-Thr-Ala-Gln-Ile-Pro-
Gln-Asp-Thr-Gly-Tyr-Gly-Gln-Ala-Tyr-His-Gly-Tyr-Trp-Gln-
Gln-Asp-Ala-Tyr-Ala-Leu-Asn-Ser-His-Tyr-Gly-Thr-Ala-Asp-
Asp-Leu-Lys-Ala-Leu-Ala-Ser-Ala-Leu-His-Ser-Arg-Gly-Met-
Tyr-Leu-Met-Val-Asp-Val-Val-Ala-Asn-His-Met-Gly-His-Asn-
Gly-Thr-Gly-Ser-Ser-Val-Asp-Tyr-Ser-Val-Tyr-Arg-Pro-Phe-
Asn-Ser-Gln-Lys-Tyr-Phe-His-Asn-Leu-Cys-Trp-Ile-Ser-Asp-
Tyr-Asn-Asn-Gln-Thr-Asn-Val-Glu-Asp-Cys-Trp-Leu-Gly-Asp-
Asn-Thr-Val-Ala-Leu-Pro-Asp-Leu-Asp-Thr-Thr-Ser-Thr-Glu-

Val-Lys-Asn-Met-Trp-Tyr-Asp-Trp-Val-Glu-Ser-Leu-Val-Ser-
Asn-Tyr-Ser-Val-Asp-Gly-Leu-Arg-Val-Asp-Thr-Val-Lys-Asn-
Val-Gln-Lys-Asn-Phe-Trp-Pro-Gly-Tyr-Asn-Asn-Ala-Ser-Gly-
Val-Tyr-Cys-Ile-Gly-Glu-Val-Phe-Asp-Gly-Asp-Ala-Ser-Tyr-
Thr-Cys-Pro-Tyr-Gln-Glu-Asp-Leu-Asp-Gly-Val-Leu-Asn-Tyr-
Pro-Met-Tyr-Tyr-Pro-Leu-Leu-Arg-Ala-Phe-Glu-Ser-Thr-Asn-
Gly-Ser-Ile-Ser-Asp-Leu-Tyr-Asn-Met-Ile-Asn-Tyr-Val-Lys-
Ser-Thr-Cys-Arg-Asp-Ser-Thr-Leu-Leu-Gly-Thr-Phe-Val-Glu-
Asn-His-Asp-Asn-Pro-Arg-Phe-Ala-Lys-Tyr-Thr-Ser-Asp-Met-
Ser-Leu-Ala-Lys-Asn-Ala-Ala-Thr-Phe-Thr-Ile-Leu-Ala-Asp-
Gly-Ile-Pro-Ile-Ile-Tyr-Ala-Gly-Gln-Glu-Gln-His-Tyr-Ser-
Gly-Gly-Asn-Asp-Pro-Tyr-Asn-Arg-Glu-Ala-Thr-Trp-Leu-Ser-
Gly-Tyr-Lys-Thr-Thr-Ser-Glu-Leu-Tyr-Thr-His-Ile-Ala-Ala-
Ser-Asn-Lys-Ile-Arg-Thr-His-Ala-Ile-Lys-Gln-Asp-Thr-Gly-
Tyr-Leu-Thr-Tyr-Lys-Asn-Tyr-Pro-Ile-Tyr-Gln-Asp-Thr-Ser-
Thr-Leu-Ala-Met-Arg-Lys-Gly-Tyr-Asn-Gly-Thr-Gln-Thr-Ile-
Thr-Val-Leu-Ser-Asn-Leu-Gly-Ala-Ser-Gly-Ser-Ser-Tyr-Thr-
Leu-Ser-Leu-Pro-Gly-Thr-Gly-Tyr-Thr-Ala-Gly-Gln-Lys-Ile-
Thr-Glu-Ile-Tyr-Thr-Cys-Thr-Asn-Leu-Thr-Val-Asn-Ser-Asn-
Gly-Ser-Val-Pro-Val-Pro-Met-Lys-Ser-Gly-Leu-Pro-Arg-Ile-
Leu-Tyr-Pro-Ala-Asp-Lys-Leu-Val-Asn-Gly-Ser-Ser-Phe-Cys-
Ser

The above amino acid sequence shows 74% homology to the TAKA-amylase enzyme.

### Example 1

### Cloning of the A. niger neutral α-amylase genes

Mycelium from A. niger DSM 2761 was harvested and processed for preparation of DNA according to the method described by Boel et al., EMBO Journal 3, 1581-85 (1984). The chromosomal DNA was cut with BamHI, EcoRI, SalI, and HindIII and analyzed by Southern blotting essentially according to Southern, J.Mol.Biol. 98, 503-18 (1975). A partial cDNA clone for TAKA-amylase was used as hybridization probe covering the first 300 amino acids of the structural gene. The TAKA-amylase cDNA clone was prepared as described in published EP patent application No. 0238023. The choice of probe is based on the similarity between TAKA-amylase from A. oryzae and the neutral α-amylase from A. niger (Minoda et al., Agr.Biol.Chem. 33 (4), 572-78 (1969)). The Southern analysis shows that A. niger has 2 genes for neutral α-amylase. For cloning we chose HindIII digestion where the 2 genes are represented by fragments of about 8.0 kb and 4.0 kb, respectively which were inserted into HindIII digested, dephosphorylated pUC8 (Vieira et al., Gene 19, 259-68 (1982)). From 5000 clones of each kind we found 1 HindIII clone of 8.0 kb and 4 HindIII clones of 4.0 kb, which hybridized with TAKA-amylase cDNA. Restriction maps of the plasmids pNA1 and pNA2 carrying the two amylase genes are shown in fig. 1. Both plasmids contain full length amylase genes with promoters and upstream activating sequences.

### Example 2

### Cloning of the gene coding for a so far undescribed amylase in A. niger

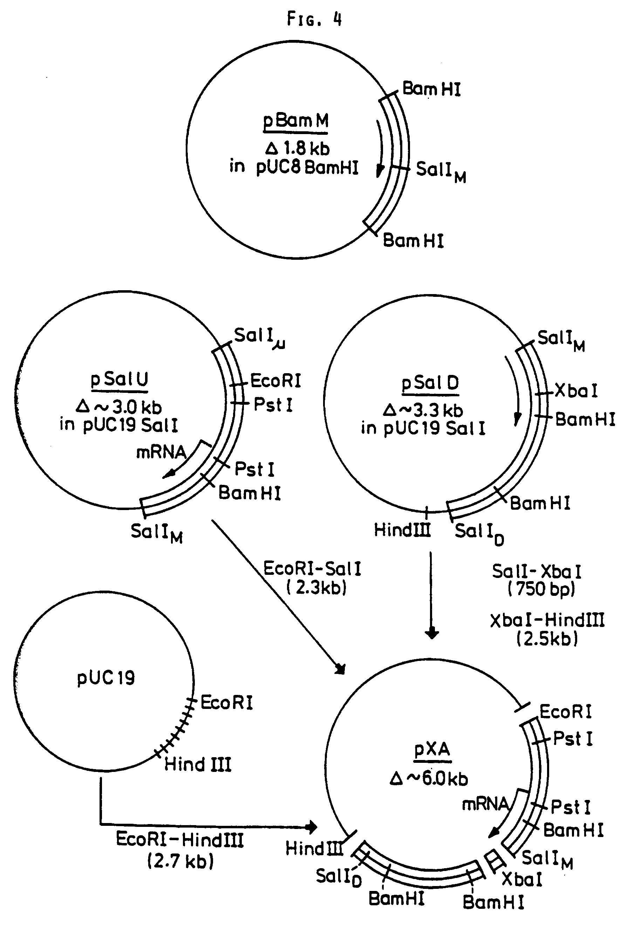

On the Southern blot described in example 1 the neutral α-amylase genes hybridized strongly to the TAKA-amylase cDNA probe. On the same blot the cDNA probe was seen to hybridize weakly to other genes, which would indicate a structural relationship to the amylase. Thus, on the basis of weak hybridization we cloned from A. niger DSM 2761 a 1.8 kb BamHI fragment into BamHI digested, dephosphorylated pUC8 and from SalI digestion we cloned fragments of 3.0 - 3.5 kb into SalI digested, dephosphorylated pUC19 (Messing, Meth. in Enzymology 101, 20-27 (1983)) There were two kinds of SalI clones which turned out to cover about half of the structural gene each. The clones covering the N-terminal have about 2.0 kb upstream of the signal sequence. The BamHI clones were found to cover parts of both types of SalI clones with the connecting SalI site almost in the middle. Fig. 4 shows the three plasmids, pBamM, pSalU and pSalD and the constructed plasmid pXA with the complete gene including promoter and upstream activating sequences. Analysis of the amino acid sequence shows 74% homology to TAKA-amylase which should leave no doubt that the cloned gene is indeed coding for an amylase. The designation for it will be XA.

The DNA-sequence of the XA-amylase promoter and upstream activating sequences, the preregion and the 5' part of the structural gene is shown in fig. 5.

### Example 3

### Cloning of the A. niger acid α-amylase gene

The Southern blot described in example 1 was hybridized to an oligonucleotide probe 
                      T      A
NOR-525+527: 5' CGCCA TCNGC GC    3'
                      C      G
 covering the N-terminal amino acids 3-7 in the acid stable α-amylase. One of the hybridizing fragments was a SalI fragment of about 3.0 kb which was cloned into SalI digested, dephosphorylated pUC19. From 20,000 clones 10 were found which hybridized to NOR-525+527. They all had the same 3.0 kb SalI insert as shown in fig. 6. Sequence analysis shows that about half of the structural gene for the acid stable α-amylase is present while promoter and upstream sequences cover about 2.0 kb.

### Example 4

### Expression of A. niger neutral α-amylase in A. oryzae

A. oryzae was used as host to analyze the potential of the A. niger neutral α-amylase promoters, as the gene product is much more stable in A. oryzae than in A. niger. Also, it is assumed that the promoters perform at least as well in their inherent host A. niger as in A. oryzae.

A. oryzae IFO 4177 was transformed with pNA1 and pNA2 respectively, using selection on acetamide by cotransformation with p3SR2 harbouring the amdS gene from A. nidulans (Tilbum, J.G. et al., Gene 26, 205-221 (1913)). Transformation was performed as described in the published EP patent application No. 0238023.

The two types of transformants were grown at 30°C in YPD medium (Sherman et al., Methods in Yeast Genetics, Cold Spring Harbor Laboratory (1981)). After 3-6 days of growth, culture supernatants were analyzed by SDS-PAGE, followed by Coomassie stain or ELISA on Western blot. The expression level of neutral α-amylase from both types of transformants was up to 10 times higher than in the untransformed IFO 4177 harbouring its own neutral α-amylase, called TAKA-amylase. In the transformants the yield of amylase was about 1 g/l supernatant thus demonstration the efficiency of the promoter and upstream activating sequences from these two genes.

### Example 5

### Expression vectors containing promoter and upstream activating sequences from A. niger amylase genes followed by the prepro-sequence of Rhizomucor miehei aspartic protease

Aspartic protease from Rhizomucor miehei (in the following called RMP) is chosen to demonstrate the production and secretion of a heterologous protein in A. niger and A. oryzae using upstream sequences from A. niger amylase genes to promote synthesis.

The 3 constructions to be outlined below have some common features. One is the use of pRMP AMG Term, the plasmid donating the RMP gene. This plasmid is described in detail in the published EP patent application No. 0238023. It has a BamHI site 9 bp upstream of the ATG initiating the preregion and following the structural gene of RMP it has a terminator sequence from the A. niger glucoamylase gene. Another feature is the use of exonuclase III → S1 nuclase → Klenow fragment, according to Henikoff, S. Gene 28, 351-59 (1984) in order to cut back (100-200 bp) from a site downstream of the initiating ATG in the amylase genes to obtain a blunt end just upstream of the ATG and thus be able to pick up a BamHI site (from pUC 19) to join to the BamHI site in pRMP AMG Term.

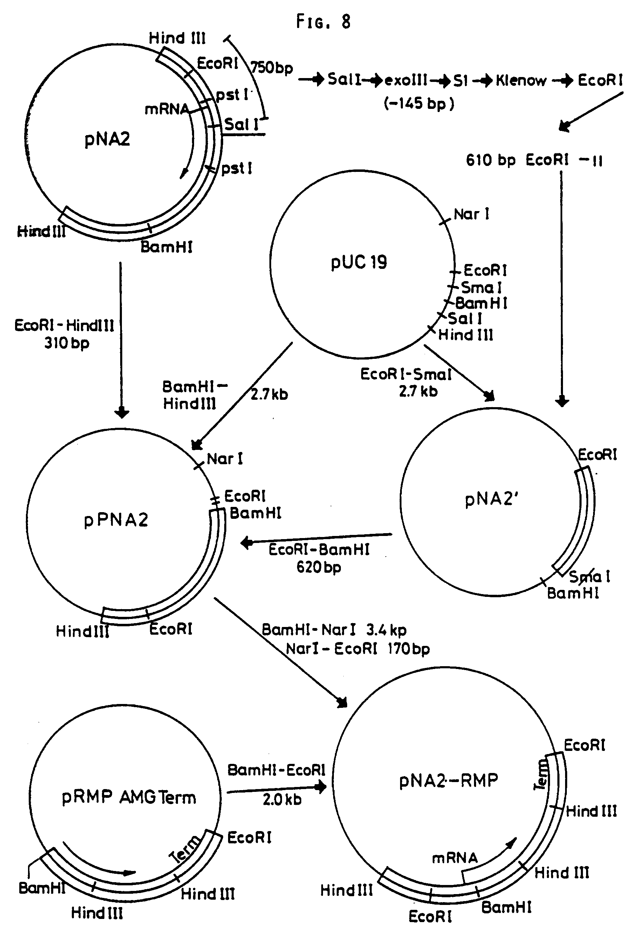

Fig. 8 illustrates the construction of the RMP gene under control of the promoter and upstream activating sequences from the neutral α-amylase gene in pNA2 (Fig. 1). Approximately 145 bp are cut back from SalI site, as verified by sequencing later, to yield an EcoRI-blunt end fragment of about 610 bp. This fragment is inserted into pUC19 cut with SmaI and EcoRI and cut out again as a 620 bp EcoRI-BamHI fragment. This fragment is ligated to a 310 bp EcoRI-HindIII fragment from upstream pNA2 and pUC19 cut with BamHI and HindIII to yield pPNA2. From this plasmid a BamHI-NarI fragment of 3.4 kb and a NarI-EcoRI fragment of 170 bp are ligated to a BamHI-EcoRI 2.0 kb from pRMP AMG Term to give the expression vector pPNA2-RMP.

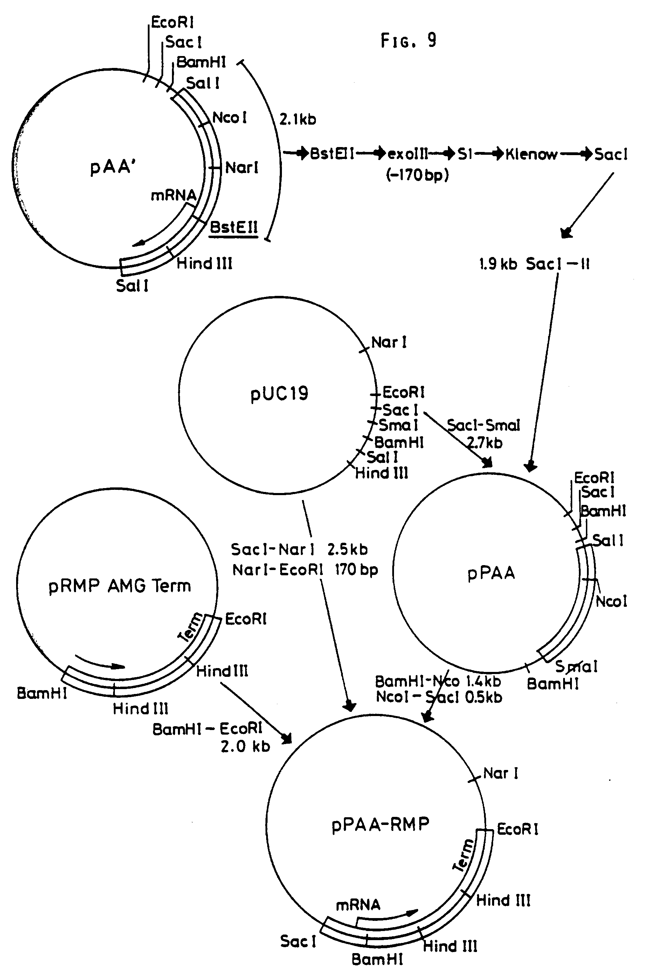

Fig. 9 shows the construction of the RMP gene under control of the promoter and upstream activating sequences from the acid amylase gene in pAA', where the gene is inserted in pUC19 in the opposite orientation of pAA (Fig. 6). About 170 bp are cut back from BstEII site to yield a fragment of 1.9 kb when cut with SacI. This SacI-blunt end fragment is inserted into pUC19 cut with SmaI and SacI and excised again as two fragments BamHI-NcoI 1.4 kb and NcoI-SacI 0.5 kb. These fragments are ligated to fragment BamHI-EcoRI 2.0 kb from pRMP AMG Term and two fragments from pUC19, SacI-NarI 2.5 kb and NarI-EcoRI 170 bp, to give the expression plasmid pPAA-RMP.

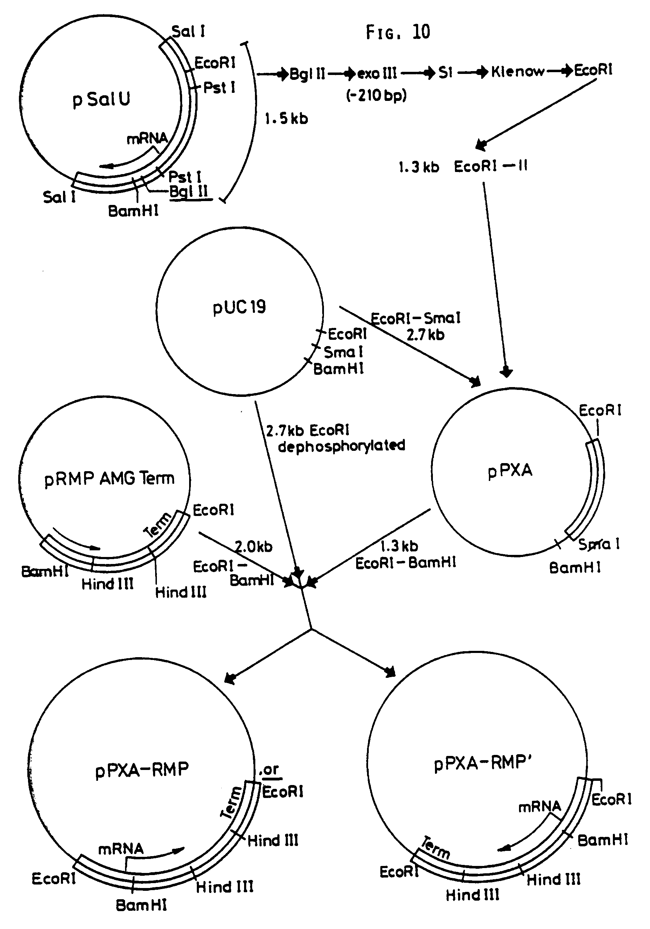

Fig. 10 outlines the construction of the RMP gene under control of the promoter and upstream activating sequences from the new amylase gene in pSalU (Fig. 4). About 210 bp are cut off from the BglII site to yield a fragment of 1.3 kb when cut with EcoRI. This fragment is inserted into pUC19 EcoRI-SmaI 2.7 kb to pick up the BamHI site next to the blunt end. The final ligation of 2.0 kb EcoRI-BamHI from pRMP AMG Term, 1.3 kb EcoIR-BamHI from pPXA and pUC19 2.7 kb EcoRI dephosphorylated fragment yields two correct expression plasmids pPXA-RMP and pPXA-RMP' with the gene in either orientation. The incorrect plasmids having 2 EcoRI-BamHI fragments of the same kind are easily discriminated by restriction analysis.

The expression plasmids are transformed into A. niger (Kelly, J.M. and Hynes, M.J., EMBO Journal 4, 475-479 (1985) and Buxton, F.P., et al., Gene 37, 207-214 (1985) using argB as selection marker and into A. oryzae as outlined above. Transformants grown in YPD are analyzed on SDS-PAGE as above and activity of the protease RMP is measured.

## Claims

1. Promoter and upstream activating sequences derived from an Aspergillus niger α-amylase gene and having the following sequence 
TCTAAACGTC GTCAAAGGTC TGTCTTCTTT CCGTATTGTC ATCTTGTAAT
ACGCTTCCTC AATGTCGTAT TTCGAAAAGA AACGGGCTTT CTTTATCCAA
TCCCTGTGGT AAGATTGATC GTCAGGAGAT TATCTGCAGG AAACATCATG
GTGGGGTAAC CAAGGTTGTG TCTGTATAAT ATATACATGT AAAATACATG
AGCTTCGGTG ATATAATACA GAAGTACCAT ACAGTACCGC GTTATGAAAA
CACATTAATC CGGATCCTTT CCTATAATAG ACTAGCGTGC TTGGCATTAG
GGTTCGAAAA ACAATCGAAG AGTATAAGGG GATGACAGCA GTAACGACTC
CAACTGTACG CCTCCGGGTA GTAGTCCGAG CAGCCGAGCC AGCTCAGCGC
CTAAAACGCC TTATACAATT AAGCAGTTAA AGAAGTTAGA ATCTACGCTT
AAAAAGCTAC TTAAAAATCG ATCTCGCAGT CCCGATTCGC CTATCAAAAC
CAGTTTAAAT CAACTGATTA AAGGTGCCGA ACGAGCTATA AATGATATAA
CAATATTAAA GCATTAATTA GAGCAATATC AGGCCGCGCA CGAAAGGCAA
CTTAAAAGCG AAAGCGCTCT ACTAAACAGA TTACTTTTGA AAAAGGCACA
TCAGTATTTA AAGCCCGAAT CCTTATTAAG CGCCGAAATC AGGCAGATAA
AGCCATACAG GCAGATAGAC CTCTACCTAT TAAATCGGCT TCTAGGCGCG
CTCCATCTAA ATGTTCTGGC TGTGGTGTAC AGGGGCATAA AATTACGCAC
TACCCGAATC GATAGAACTA CTCATTTTTA TATAGAAGTC AGAATTCATG
GTGTTTTGAT CATTTTAAAT TTTTATATGG CGGGTGGTGG GCAACTCGCT
TGCGCGGCAA CTCGCTTACC GATTACGTTA GGGCTGATAT TTACGTAAAA
ATCGTCAAGG GATGCAAGAC CAAAGTAGTA AAACCCCGGA GTCAACAGCA
TCCAAGCCCA AGTCCTTCAC GGAGAAACCC CAGCGTCCAC ATCACGAGCG
AAGGACCACC TCTAGGCATC GGACGCACCA TCCAATTAGA AGCAGCAAAG
CGAAACAGCC CAAGAAAAAG GTCGGCCCGT CGGCCTTTTC TGCAACGCTG
ATCACGGGCA GCGATCCAAC CAACACCCTC CAGAGTGACT AGGGGCGGAA
ATTTAAAGGG ATTAATTTCC ACTCAACCAC AAATCACAGT CGTCCCCGGT
ATTGTCCTGC AGAATGCAAT TTAAACTCTT CTGCGAATCG CTTGGATTCC
CCGCCCCTAG CGTAGAGCTT AAAGTATGTC CCTTGTCGAT GCGATGTATC
ACAACATATA AATACTAGCA AGGGATGCCA TGCTTGGAGG ATAGCAACCG
ACAACATCAC ATCAAGCTCT CCCTTCTCTG AACAATAAAC CCCACAGAAG
GCATTT
 or functional fragments thereof, or having the following sequence 
AAGCTTCCAG CTACCGTAGA TTACTGATAC AAACTCAATA CACTATTTCT
ATAACCTTAC TGTTCAATAC AGTACGATCA AAATTTCCGG AATATTAATG
TTACGGTTAC CTTCCATATG TAGACTAGCG CACTTGGCAT TAGGGTTCGA
AATACGATCA AAGAGTATTG GGGGGGTGA CAGCAGTAAT GACTCCAACT
GTAAATCGGC TTCTAGGCGC GCTCCATCTA AATGTTCTGG CTGTGGTGTA
CAGGGGCATA AAATTACGCA CTACCCGAAT CGATAGAACT ACTCATTTTT
ATATAGAAGT CAGAATTCAT GGTGTTTTGA TCATTTTAAA TTTTTATATG
GCGGGTGGTG GGCAACTCGC TTGCGCGGCA ACTCGCTTAC CGATTACGTT
AGGGCTGATA TTTACGTAAA AATCGTCAAG GGATGCAAGA CCAAAGTACT
AAAACCCCGG AGTCAACAGC ATCCAAGCCC AAGTCCTTCA CGGAGAAACC
CCAGCGTCCA CATCACGAGC GAAGGACCAC CTCTAGGCAT CGGACGCACC
ATCCAATTAG AAGCAGCAAA GCGAAACAGC CCAAGAAAAA GGTCGGCCCG
TCGGCCTTTT CTGCAACGCT GATCACGGGC AGCGATCCAA CCAACACCCT
CCAGAGTGAC TAGGGGCGGA AATTTATCGG GATTAATTTC CACTCAACCA
CAAATCACAG TCGTCCCCGG TATTGTCCTG CAGAATGCAA TTTAAACTCT
TCTGCGAATC GCTTGGATTC CCCGCCCCTA GCGTAGAGCT TAAAGTATGT
CCCTTGTCGA TGCGATGTAT CACAACATAT AAATACTAGC AAGGATGCC
ATGCTTGGAG GATAGCAACC GACAACATCA CATCAAGCTC TCCCTTCTCT
GAACAATAAA CCCCACAGAA GGCATTT
 or functional fragments thereof, or having the following sequence 
CCTAATGACC CAACATTGGC TGCGGTTGAG ACTCAATTCA TGGTTGGGCC
GGCCATCATG GTGGTCCCGG TATTGGAGCC TCTGGTCAAT ACGGTCAAGG
GCGTATTCCC AGGAGTTGGA CATGGCGAAG TGTGGTACGA TTGGTACACC
CAGGCTGCAG TTGATGCGAA GCCCGGGGTC AACACGACCA TTTCGGCACC
ATTGGGCCAC ATCCCAGTTT ATGTACGAGG TGGAAACATC TTGCCGATGC
AAGAGCCGGC ATTGACCACT CGTGAAGCCC GGCAAACCCC GTGGGCTTTG
CTAGCTGCAC TAGGAAGCAA TGGAACCGCG TCGGGGCAGC TCTATCTCGA
TGATGGAGAG AGCATCTACC CCAATGCCAC CCTCCATGTG GACTTCACGG
CATCGCGGTC AAGCCTGCGC TCGTCGGCTC AAGGAAGATG GAAAGAGAGG
AACCCGCTTG CTAATGTGAC GGTGCTCGGA GTGAACAAGG TGCCCTCTGC
GGTGACCCTG AATGGACAGG CCGTATTTCC CGGGTCTGTC ACGTACAATT
 
CTACGTCCCA GGTTCTCTTT GTTGGGGGGC TGCAAAACTT GACGAAGGGC
GGCGCATGGG CGGAAAACTG GGTATTGGAA TGGTAGTGTC AGCCACAAGC
CAGGTGTGCG CGTACAGCAT GCAACATGGG AACGATGCTC TGCAATGTAG
CTCTTTGGTT ATAATTCAAA ATTCAACTTC CACCTTTGTT TCACCGGCGG
CCACGGCATT CCTGCATGAC TAACGTTCTG TAAATGGACC CGATAACACC
CAGCACGTTG CAGCAGAGAA GGTACTCTCT CACACGCACT GCTCTTTATA
GTTGCCGAGA CGGCCGCCGA GGAGAAAACC GCCGGCCTGT GGCCACTATT
CGCTGGAAGG AACCCTGCCA GTCGAACACA CCCGCCCGTG ATCGCCAGGG
GCCGATGGAT TTCCCCCCGC ATCCTTGTCG GTTCATGAGT GAAGACTTTA
AATCCCATCT AGCTGACGGT CGGGTACATC AATAACTGGC GGCCTGGTTT
CCAGGACACG GAGAGGCATC TAATCGCTAT TTATAGAATG CTGGGATCGG
ACCCGTCGAA TGGTCTTCCG ATGGGAAGTG ACAACTCACA TTGTCATGTT
GGCCTTACTC AATCCAACGG GATCTGACCT GCTTTGGCTA ACCTAGTATA
AATCAGCATG TCTCTCCTTT GATACATCGG ATCGTTCCTC AAATATAGTT
ATATCTTCGA AAAATTGACA AGAAGG
 or functional fragments thereof.

2. A process for expression of a protein product in Aspergillus comprising the steps of:
(a) providing a recombinant DNA cloning vector system capable of integration into the genome of an Aspergillus host in one or more copies and comprising: DNA-sequences encoding an Aspergillus niger amylase promoter and upstream activating sequences according to claim 1; a suitable marker for selection of transformants; and a DNA-sequence encoding the desired protein product;
(b) transforming the Aspergillus host which does not harbour a functional gene for the chosen selection marker with the recombinant DNA cloning vector system from step a; and
c) culturing the transformed Aspergillus host in a suitable culture medium.

3. A process according to claim 2, wherein the host is an Aspergillus niger strain.

4. A process according to claim 2, wherein the selection marker is derived from the gene for A. nidulans or A. niger argB. A. nidulans trpC, A. nidulans amdS, Neurospora crassae Pyr4 or DHFR.

5. A process according to claim 4, wherein the selection marker is the ArgB gene derived from A. nidulans or A. niger or the amdS gene derived from A. nidulans.

6. A process according to claim 2, wherein the vector system further comprises a preregion providing for secretion of the expressed product into the culture medium.

7. A process according to claim 6, wherein the preregion is derived from a glucoamylase or an amylase gene from an Aspergillus species, an amylase gene from a Bacillus species, a lipase or proteinase gene from Rhizomucor miehei, the gene for the α-factor from S. cerevisiae, the calf prochymosin gene or the gene for the desired protein.

8. A process according to claim 7, wherein the preregion is derived from the gene for A. oryzae TAKA amylase, A. niger neutral α-amylase, A. niger acid-stable α-amylase, B. licheniformis α-amylase, the preregion from the A. niger XA amylase as described in example 2 of the description, the maltogenic amylase from Bacillus NCIB 11837, B. stearothermophilus α-amylase or B. licheniformis subtilisin.

9. A process according to claim 8, wherein the preregion is the TAKA-amylase preregion or the A. niger neutral α-amylase preregion with the following sequence:

ATGATGGTCGCGTGGTGGTCTCTATTTCTGTACGGCCTTCAGGTCGCGGCACCT
MetMetValAlaTrpTrpSerLeuPheLeuTyrGlyLeuGlnValAlaAlaPro

GCTTTGGCT
AlaLeuAla

10. A process according to claim 8, wherein the preregion is the A. niger acid stable α-amylase preregion with the following sequence:

ATGAGATTATCGACTTCGAGTCTCTTCCTTTCCGTGTCTCTGCTGGGGAAGCTGGCC
MetArgLeuSerThrSerSerLeuPheLeuSerValSerLeuLeuGlyLysLeuAla

CTCGGG
LeuGly

11. A process according to claim 8, wherein the preregion is the XA niger amylase preregion with the sequence:

ATGACAATCTTTCTGTTTCTGGCCATTTTCGTGGCTACAGCTCTGGCA
MetThrIlePheLeuPheLeuAlaIlePheValAlaThrAlaLeuAla

12. A process according to claim 2, wherein the vector system comprises two vectors, where one contains the selection marker and the other contains DNA-sequences encoding functions facilitating gene expression and a DNA-sequence encoding the desired protein product.

13. A process for production of a protein product in Aspergillus, wherein an Aspergillus strain being transformed with a recombinant DNA cloning vector system as described in claim 2 is cultured in a suitable culture medium and the product is recovered from the culture medium.

14. A process according to claim 13, wherein the Aspergillus strain is an Aspergillus niger strain.

## Patentansprüche

1. Promotor- und Flußaufwärts-Aktivator-Sequenzen, abgeleitet von einem Aspergillus niger-α-Amylase-Gen und mit der folgenden Sequenz 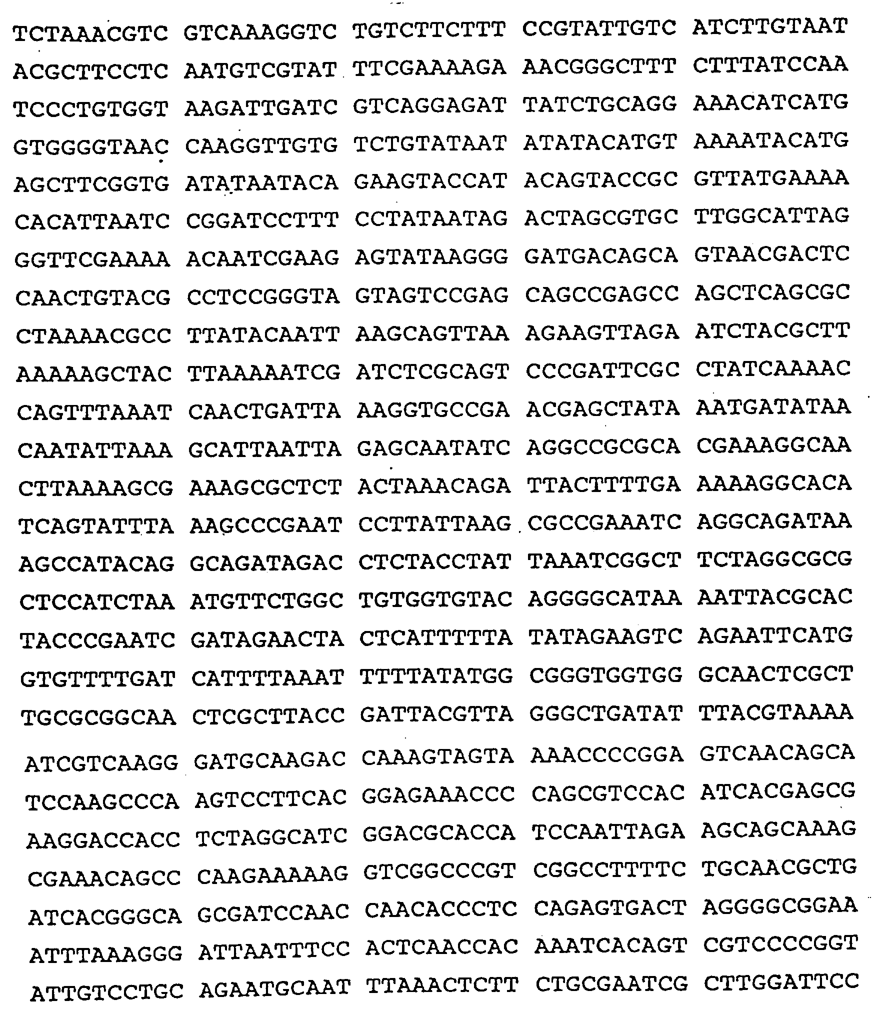 
CCGCCCCTAG CGTAGAGCTT AAAGTATGTC CCTTGTCGAT GCGATGTATC
ACAACATATA AATACTAGCA AGGGATGCCA TGCTTGGAGG ATAGCAACCG
ACAACATCAC ATCAAGCTCT CCCTTCTCTG AACAATAAAC CCCACAGAAG
GCATTT
 oder funktionelle Fragmente derselben, oder mit der folgenden Sequenz 
AAGCTTCCAG CTACCGTAGA TTACTGATAC AAACTCAATA CACTATTTCT
ATAACCTTAC TGTTCAATAC AGTACGATCA AAATTTCCGG AATATTAATG
TTACGGTTAC CTTCCATATG TAGACTAGCG CACTTGGCAT TAGGGTTCGA
AATACGATCA AAGAGTATTG GGGGGGGTGA CAGCAGTAAT GACTCCAACT
GTAAATCGGC TTCTAGGCGC GCTCCATCTA AATGTTCTGG CTGTGGTGTA
CAGGGGCATA AAATTACGCA CTACCCGAAT CGATAGAACT ACTCATTTTT
ATATAGAAGT CAGAATTCAT GGTGTTTTGA TCATTTTAAA TTTTTATATG
GCGGGTGGTG GGCAACTCGC TTGCGCGGCA ACTCGCTTAC CGATTACGTT
AGGGCTGATA TTTACGTAAA AATCGTCAAG GGATGCAAGA CCAAAGTACT
AAAACCCCGG AGTCAACAGC ATCCAAGCCC AAGTCCTTCA CGGAGAAACC
CCAGCGTCCA CATCACGAGC GAAGGACCAC CTCTAGGCAT CGGACGCACC
ATCCAATTAG AAGCAGCAAA GCGAAACAGC CCAAGAAAAA GGTCGGCCCG
TCGGCCTTTT CTGCAACGCT GATCACGGGC AGCGATCCAA CCAACACCCT
CCAGAGTGAC TAGGGGCGGA AATTTATCGG GATTAATTTC CACTCAACCA
CAAATCACAG TCGTCCCCGG TATTGTCCTG CAGAATGCAA TTTAAACTCT
TCTGCGAATC GCTTGGATTC CCCGCCCCTA GCGTAGAGCT TAAAGTATGT
CCCTTGTCGA TGCGATGTAT CACAACATAT AAATACTAGC AAGGATGCCC
ATGCTTGGAG GATAGCAACC GACAACATCA CATCAAGCTC TCCCTTCTCT
GAACAATAAA CCCCACAGAA GGCATTT
oder funktionellen Fragmente derselben, oder mit der folgenden Sequenz
CCTAATGACC CAACATTGGC TGCGGTTGAG ACTCAATTCA TGGTTGGGCC
GGCCATCATG GTGGTCCCGG TATTGGAGCC TCTGGTCAAT ACGGTCAAGG
GCGTATTCCC AGGAGTTGGA CATGGCGAAG TGTGGTACGA TTGGTACACC
CAGGCTGCAG TTGATGCGAA GCCCGGGGTC AACACGACCA TTTCGGCACC
ATTGGGCCAC ATCCCAGTTT ATGTACGAGG TGGAAACATC TTGCCGATGC
AAGAGCCGGC ATTGACCACT CGTGAAGCCC GGCAAACCCC GTGGGCTTTG
CTAGCTGCAC TAGGAAGCAA TGGAACCGCG TCGGGGCAGC TCTATCTCGA
TGATGGAGAG AGCATCTACC CCAATGCCAC CCTCCATGTG GACTTCACGG
CATCGCGGTC AAGCCTGCGC TCGTCGGCTC AAGGAAGATG GAAAGAGAGG
AACCCGCTTG CTAATGTGAC GGTGCTCGGA GTGAACAAGG TGCCCTCTGC
 
GGTGACCCTG AATGGACAGG CCGTATTTCC CGGGTCTGTC ACGTACAATT
CTACGTCCCA GGTTCTCTTT GTTGGGGGGC TGCAAAACTT GACGAAGGGC
GGCGCATGGG CGGAAAACTG GGTATTGGAA TGGTAGTGTC AGCCACAAGC
CAGGTGTGCG CGTACAGCAT GCAACATGGG AACGATGCTC TGCAATGTAG
CTCTTTGGTT ATAATTCAAA ATTCAACTTC CACCTTTGTT TCACCGGCGG
CCACGGCATT CCTGCATGAC TAACGTTCTG TAAATGGACC CGATAACACC
CAGCACGTTG CAGCAGAGAA GGTACTCTCT CACACGCACT GCTCTTTATA
GTTGCCGAGA CGGCCGCCGA GGAGAAAACC GCCGGCCTGT GGCCACTATT
CGCTGGAAGG AACCCTGCCA GTCGAACACA CCCGCCCGTG ATCGCCAGGG
GCCGATGGAT TTCCCCCCGC ATCCTTGTCG GTTCATGAGT GAAGACTTTA
AATCCCATCT AGCTGACGGT CGGGTACATC AATAACTGGC GGCCTGGTTT
CCAGGACACG GAGAGGCATC TAATCGCTAT TTATAGAATG CTGGGATCGG
ACCCGTCGAA TGGTCTTCCG ATGGGAAGTG ACAACTCACA TTGTCATGTT
GGCCTTACTC AATCCAACGG GATCTGACCT GCTTTGGCTA ACCTAGTATA
AATCAGCATG TCTCTCCTTT GATACATCGG ATCGTTCCTC AAATATAGTT
ATATCTTCGA AAAATTGACA AGAAGG
 oder funktionelle Fragmente derselben.

2. Verfahren zur Expression eines Proteinproduktes in Aspergillus, welches die Schritte umfaßt:
(a) Bereitstellen eines rekombinanten DNA-Klonierungsvektorsystems, das zur Integration in das Genom eines Aspergillus-Wirts in einer oder mehreren Kopien in der Lage ist und welches: DNASequenzen, die einen Aspergillus niger-Amylase-Promotor und -Flußaufwärts-Aktivator-Sequenzen gemaß Anspruch 1 kodieren; einen geeigneten Marker zur Selektion von Transformanten; und eine DNA-Sequenz, die das gewünschte Protein im Produkt kodiert, umfaßt;
(b) Transformieren des Aspergillus-Wirtes, der kein funktionelles Gen für den ausgewählten Selektionsmarker enthält, mit dem rekombinanten DNA-Klonierungsvektorsystem aus Schritt a;
(c) Kultivieren des transformierten Aspergillus-Wirts in einem geeigneten Kulturmedium.

3. Verfahren nach Anspruch 2, wobei der Wirt ein Aspergillus niger-Stamm ist.

4. Verfahren nach Anspruch 2, wobei der Selektionsmarker abgeleitet ist von dem Gen für A. nidulans- oder A. niger-argB, A. nidulans-trpC, A. nidulans-amdS, Neurospora crassae-Pyr4 oder DHFR.

5. Verfahren nach Anspruch 4, wobei der Selektionsmarker das argB-Gen, abgeleitet von A. nidulans oder A. niger, oder das amdS-Gen, abgeleitet von A. nidulans, ist.

6. Verfahren nach Anspruch 2, wobei das Vektorsystem auberdem eine Vorregion umfaßt, die für Sekretion des exprimierten Produktes in das Kulturmedium sorgt.

7. Verfahren nach Anspruch 6, wobei die Vorregion abgeleitet ist von einem Glucoamylase- odereinem Amylase-Gen aus einer Aspergillus-Spezies, einem Amylase-Gen aus einer Bacillus-Spezies, einem Lipase- oder Proteinase-Gen aus Rhizomucor miehei, dem Gen für den α-Faktor aus S. cerevisiae, dem Kalbs-Prochymosin-Gen oder dem Gen für das gewünschte Produkt.

8. Verfahren nach Anspruch 7, wobei die Vorregion abgeleitet ist von dem Gen für A. oryzae-TAKA-Amylase, neutrale α-Amylase aus A. niger, säurestabile α-Amylase aus A. niger, B. licheniformis-α-Amylase, die Vorregion aus der A. niger-XA-Amylase, wie beschrieben in Beispiel 2 der Beschreibung, die maltogene Amylase aus Bacillus NCIB 11837, B. stearothermophilus-α-Amylase oder B. licheniformis subtilisin.

9. Verfahren nach Anspruch 8, wobei die Vorregion die TAKA-Amylase-Vorregion oder die Vorregion der neutralen α-Amylase aus A. niger ist mit der folgenden Sequenz;

ATGATGGTCGCGTGGTGGTCTCTATTTCTGTACGGCCTTCAGGTCGCGGCACCT
MetMetValAlaTrpTrpSerLeuPheLeuTyrGlyLeuGlnValAlaAlaPro

GCTTTGGCT
AlaLeuAla

10. Verfahren nach Anspruch 8, wobei die Vorregion die Vorregion der säurestabilen α-Amylase aus A. niger ist mit der folgenden Sequenz:

ATGAGATTATCGACTTCGAGTCTCTTCCTTTCCGTGTCTCTGCTGGGGAAGCTGGCC
MetArgLeuSerThrSerSerLeuPheLeuSerValSerLeuLeuGlyLysLeuAla

CTCGGG
LeuGly

11. Verfahren nach Anspruch 8, wobei die Vorregion die XA-niger-Amylase-Vorregion ist mit der Sequenz:

ATGACAATCTTTCTGTTTCTGGCCATTTTCGTGGCTACAGCTCTGGCA
MetThrIlePheLeuPheLeuAlaIlePheValAlaThrAlaLeuAla

12. Verfahren nach Anspruch 2, wobei das Vektorsystem zwei Vektoren umfaßt, wobei einer den Selektionsmarker enthält und der andere DNA-Sequenzen enthält, die Funktionen kodieren, die Genexpression erleichtert, und eine DNA-Sequenz, die das gewünschte Proteinprodukt kodiert.

13. Verfahren zur Herstellung eines Proteinproduktes in Aspergillus, wobei ein Aspergillus-Stamm, der mit einem rekombinanten DNA-Klonierungsvektorsystem, wie in Anspruch 2 beschrieben, transformiert worden ist, in einem geeigneten Kulturmedium kultiviert wird und das Produkt aus dem Kulturmedium gewonnen wird.

14. Verfahren nach Anspruch 13, wobei der Aspergillus-Stamm ein Aspergillus niger-Stamm ist.

## Revendications

1. Séquences du promoteur et d'activation en amont dérivées d'un gène d'α-amylase d'Aspergillus niger et ayant la séquence suivante: 
TCTAAACGTC GTCAAAGGTC TGTCTTCTTT CCGTATTGTC ATCTTGTAAT
ACGCTTCCTC AATGTCGTAT TTCGAAAAGA AACGGGCTTT CTTTATCCAA
TCCCTGTGGT AAGATTGATC GTCAGGAGAT TATCTGCAGG AAACATCATG
GTGGGGTAAC CAAGGTTGTG TCTGTATAAT ATATACATGT AAAATACATG
AGCTTCGGTG ATATAATACA GAAGTACCAT ACAGTACCGC GTTATGAAAA
CACATTAATC CGGATCCTTT CCTATAATAG ACTAGCGTGC TTGGCATTAG
GGTTCGAAAA ACAATCGAAG AGTATAAGGG GATGACAGCA GTAACGACTC
CAACTGTACG CCTCCGGGTA GTAGTCCGAG CAGCCGAGCC AGCTCAGCGC
CTAAAACGCC TTATACAATT AAGCAGTTAA AGAAGTTAGA ATCTACGCTT
AAAAAGCTAC TTAAAAATCG ATCTCGCAGT CCCGATTCGC CTATCAAAAC
CAGTTTAAAT CAACTGATTA AAGGTGCCGA ACGAGCTATA AATGATATAA
CAATATTAAA GCATTAATTA GAGCAATATC AGGCCGCGCA CGAAAGGCAA
CTTAAAAGCG AAAGCGCTCT ACTAAACAGA TTACTTTTGA AAAAGGCACA
TCAGTATTTA AAGCCCGAAT CCTTATTAAG CGCCGAAATC AGGCAGATAA
AGCCATACAG GCAGATAGAC CTCTACCTAT TAAATCGGCT TCTAGGCGCG
CTCCATCTAA ATGTTCTGGC TGTGGTGTAC AGGGGCATAA AATTACGCAC
TACCCGAATC GATAGAACTA CTCATTTTTA TATAGAAGTC AGAATTCATG
GTGTTTTGAT CATTTTAAAT TTTTATATGG CGGGTGGTGG GCAACTCGCT
TGCGCGGCAA CTCGCTTACC GATTACGTTA GGGCTGATAT TTACGTAAAA
ATCGTCAAGG GATGCAAGAC CAAAGTAGTA AAACCCCGGA GTCAACAGCA
TCCAAGCCCA AGTCCTTCAC GGAGAAACCC CAGCGTCCAC ATCACGAGCG
AAGGACCACC TCTAGGCATC GGACGCACCA TCCAATTAGA AGCAGCAAAG
CGAAACAGCC CAAGAAAAAG GTCGGCCCGT CGGCCTTTTC TGCAACGCTG
ATCACGGGCA GCGATCCAAC CAACACCCTC CAGAGTGACT AGGGGCGGAA
ATTTAAAGGG ATTAATTTCC ACTCAACCAC AAATCACAGT CGTCCCCGGT
ATTGTCCTGC AGAATGCAAT TTAAACTCTT CTGCGAATCG CTTGGATTCC
CCGCCCCTAG CGTAGAGCTT AAAGTATGTC CCTTGTCGAT GCGATGTATC
ACAACATATA AATACTAGCA AGGGATGCCA TGCTTGGAGG ATAGCAACCG
ACAACATCAC ATCAAGCTCT CCCTTCTCTG AACAATAAAC CCCACAGAAG
GCATTT
 ou des fragments fonctionnels de cette séquence, ou ayant la séquence suivante: 
AAGCTTCCAG CTACCGTAGA TTACTGATAC AAACTCAATA CACTATTTCT
ATAACCTTAC TGTTCAATAC AGTACGATCA AAATTTCCGG AATATTAATG
TTACGGTTAC CTTCCATATG TAGACTAGCG CACTTGGCAT TAGGGTTCGA
AATACGATCA AAGAGTATTG GGGGGGGTGA CAGCAGTAAT GACTCCAACT
GTAAATCGGC TTCTAGGCGC GCTCCATCTA AATGTTCTGG CTGTGGTGTA
CAGGGGCATA AAATTACGCA CTACCCGAAT CGATAGAACT ACTCATTTTT
ATATAGAAGT CAGAATTCAT GGTGTTTTGA TCATTTTAAA TTTTTATATG
GCGGGTGGTG GGCAACTCGC TTGCGCGGCA ACTCGCTTAC CGATTACGTT
AGGGCTGATA TTTACGTAAA AATCGTCAAG GGATGCAAGA CCAAAGTACT
AAAACCCCGG AGTCAACAGC ATCCAAGCCC AAGTCCTTCA CGGAGAAACC
CCAGCGTCCA CATCACGAGC GAAGGACCAC CTCTAGGCAT CGGACGCACC
ATCCAATTAG AAGCAGCAAA GCGAAACAGC CCAAGAAAAA GGTCGGCCCG
TCGGCCTTTT CTGCAACGCT GATCACGGGC AGCGATCCAA CCAACACCCT
CCAGAGTGAC TAGGGGCGGA AATTTATCGG GATTAATTTC CACTCAACCA
CAAATCACAG TCGTCCCCGG TATTGTCCTG CAGAATGCAA TTTAAACTCT
TCTGCGAATC GCTTGGATTC CCCGCCCCTA GCGTAGAGCT TAAAGTATGT
CCCTTGTCGA TGCGATGTAT CACAACATAT AAATACTAGC AAGGATGCC
ATGCTTGGAG GATAGCAACC GACAACATCA CATCAAGCTC TCCCTTCTCT
GAACAATAAA CCCCACAGAA GGCATTT
 ou des fragments fonctionnels de cette séquence, ou ayant la séquence suivante: 
CCTAATGACC CAACATTGGC TGCGGTTGAG ACTCAATTCA TGGTTGGGCC
GGCCATCATG GTGGTCCCGG TATTGGAGCC TCTGGTCAAT ACGGTCAAGG
GCGTATTCCC AGGAGTTGGA CATGGCGAAG TGTGGTACGA TTGGTACACC
CAGGCTGCAG TTGATGCGAA GCCCGGGGTC AACACGACCA TTTCGGCACC
ATTGGGCCAC ATCCCAGTTT ATGTACGAGG TGGAAACATC TTGCCGATGC
AAGAGCCGGC ATTGACCACT CGTGAAGCCC GGCAAACCCC GTGGGCTTTG
CTAGCTGCAC TAGGAAGCAA TGGAACCGCG TCGGGGCAGC TCTATCTCGA
TGATGGAGAG AGCATCTACC CCAATGCCAC CCTCCATGTG GACTTCACGG
CATCGCGGTC AAGCCTGCGC TCGTCGGCTC AAGGAAGATG GAAAGAGAGG
AACCCGCTTG CTAATGTGAC GGTGCTCGGA GTGAACAAGG TGCCCTCTGC
GGTGACCCTG AATGGACAGG CCGTATTTCC CGGGTCTGTC ACGTACAATT
CTACGTCCCA GGTTCTCTTT GTTGGGGGGC TGCAAAACTT GACGAAGGGC
 
GGCGCATGGG CGGAAAACTG GGTATTGGAA TGGTAGTGTC AGCCACAAGC
CAGGTGTGCG CGTACAGCAT GCAACATGGG AACGATGCTC TGCAATGTAG
CTCTTTGGTT ATAATTCAAA ATTCAACTTC CACCTTTGTT TCACCGGCGG
CCACGGCATT CCTGCATGAC TAACGTTCTG TAAATGGACC CGATAACACC
CAGCACGTTG CAGCAGAGAA GGTACTCTCT CACACGCACT GCTCTTTATA
GTTGCCGAGA CGGCCGCCGA GGAGAAAACC GCCGGCCTGT GGCCACTATT
CGCTGGAAGG AACCCTGCCA GTCGAACACA CCCGCCCGTG ATCGCCAGGG
GCCGATGGAT TTCCCCCCGC ATCCTTGTCG GTTCATGAGT GAAGACTTTA
AATCCCATCT AGCTGACGGT CGGGTACATC AATAACTGGC GGCCTGGTTT
CCAGGACACG GAGAGGCATC TAATCGCTAT TTATAGAATG CTGGGATCGG
ACCCGTCGAA TGGTCTTCCG ATGGGAAGTG ACAACTCACA TTGTCATGTT
GGCCTTACTC AATCCAACGG GATCTGACCT GCTTTGGCTA ACCTAGTATA
AATCAGCATG TCTCTCCTTT GATACATCGG ATCGTTCCTC AAATATAGTT
ATATCTTCGA AAAATTGACA AGAAGG
 ou des fragments fonctionnels de cette séquence.

2. Procédé pour l'expression d'un produit protéinique dans Aspergillus, comprenant les étapes de:
(a) fourniture d'un système de vecteur de clonage d'ADN recombiné capable de s'intégrer dans le génome d'un hôte Aspergillus en une ou plusieurs copies et comprenant: des séquences d'ADN codant pour des séquences de promoteur et d'activation en amont d'une amylase d'Aspergillus niger selon la revendication 1, un marqueur convenable pour la sélection des cellules transformées; et une séquence d'ADN codant pour le produit protéinique désiré;
(b) transformation de l'hôte Aspergillus, qui n'héberge pas de gène fonctionnel pour le marqueur de sélection choisi, avec le système de vecteur de clonage d'ADN recombiné de l'étape a; et
c) culture de l'hôte Aspergillus transformé dans un milieu de culture convenable.

3. Procédé selon la revendication 2, dans lequel l'hôte est une souche d'Aspergillus niger.

4. Procédé selon la revendication 2, dans lequel le marqueur de sélection est dérivé du gène pour l'argB d'A. nidulans ou d'A. niger, le trpC d'A. nidulans, l'amdS d'A. nidulans, le pyr4 de Neurospora crassae ou le DHFR.

5. Procédé selon la revendication 4, dans lequel le marqueur de sélection est le gène argB dérivé d'A. nidulans ou le gène amdS dérivé d'A. nidulans.

6. Procédé selon la revendication, 2, dans lequel le système de vecteur comprend en outre une prérégion assurant la sécrétion du produit exprimé dans le milieu de culture.

7. Procédé selon la revendication 6, dans lequel la prérégion est dérivée d'un gène de glucoamylase ou d'amylase d'une espèce d'Aspergillus, d'un gène d'amylase d'une espèce de Bacillus, d'un gène de lipase ou de protéinase de Rhizomucor miehei, du gène codant pour le facteur α de S. cerevisiae, du gène de la prochymosine de veau ou du gène pour la protéine désirée.

8. Procédé selon la revendication 7, dans lequel la prérégion est dérivée du gène pour l'amylase TAKA d'A. oryzae , l'α-amylase neutre d'A. niger, l'α-amylase stable aux acides d'A. niger. l'α-amylase de B. licheniformis, la prérégion de l'amylase XA d'A. niger telle que décrite dans l'exemple 2 de la description, l'amylase maltogène de Bacillus NCIB 11837, l'α-amylase de B. stearothermophilus ou la subtilisine de B. licheniformis.

9. Procédé selon la revendication 8, dans lequel la prérégion est la prérégion de l'amylase TAKA ou la prérégion de l'α-amylase neutre d'A. niger ayant la séquence suivante:

ATGATGGTCGCGTGGTGGTCTCTATTTCTGTACGGCCTTCAGGTCGCGGCACCT
MetMetValAlaTrpTrpSerLeuPheLeuTyrGlyLeuGlnValAlaAlaPro

GCTTTGGCT
AlaLeuAla

10. Procédé selon la revendication 8, dans lequel la prérégion est la prérégion de l'α-amylase stable aux acides d'A. niger ayant la séquence suivante:

ATGAGATTATCGACTTCGAGTCTCTTCCTTTCCGTGTCTCTGCTGGGGAAGCTGGCC
MetArgLeuSerThrSerSerLeuPheLeuSerValSerLeuLeuGlyLysLeuAla

CTCGGG
LeuGly

11. Procédé selon la revendication 8, dans lequel la prérégion est la prérégion de l'amylase XA d'A. niger ayant la séquence:

ATGACAATCTTTCTGTTTCTGGCCATTTTCGTGGCTACAGCTCTGGCA
MetThrIlePheLeuPheLeuAlaIlePheValAlaThrAlaLeuAla

12. Procédé selon la revendication 2, dans lequel le système de vecteur comprend deux vecteurs, dont l'un contient le marqueur de sélection et l'autre contient les séquences d'ADN codant pour des fonctions facilitant l'expression du gène et une séquence d'ADN codant pour le produit protéinique désiré.

13. Procédé de production d'un produit protéinique dans Aspergillus, dans lequel on cultive une souche d'Aspergillus transformée avec un système de vecteur de clonage d'ADN recombiné tel que décrit dans la revendication 2, dans un milieu de culture convenable et on récupère le produit à partir du milieu de culture.

14. Procédé selon la revendication 13, dans lequel la souche d'Aspergillus est une souche d'Aspergillus niger.
